(19) Europäisches Patentamt
European Patent Office
Office européen des brevets

(11) **EP 3 647 780 B1**

(12) **EUROPÄISCHE PATENTSCHRIFT**

(45) Veröffentlichungstag und Bekanntmachung des Hinweises auf die Patenterteilung:
**18.08.2021   Patentblatt 2021/33**

(51) Int Cl.:
*G01N 33/00* *(2006.01)*    *G01N 27/409* *(2006.01)*
*G01N 27/419* *(2006.01)*

(21) Anmeldenummer: **19204877.5**

(22) Anmeldetag: **23.10.2019**

(54) **VERFAHREN UND MESSSYSTEM ZUR ERFASSUNG EINES TEERGEHALTS IN GASEN**

METHOD AND MEASURING SYSTEM FOR DETECTING A TAR CONTENT IN GASES

PROCÉDÉ ET SYSTÈME DE MESURE PERMETTANT DE DÉTECTER UN TENEUR EN GOUDRON DANS DES GAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.10.2018   DE 102018126467**

(43) Veröffentlichungstag der Anmeldung:
**06.05.2020   Patentblatt 2020/19**

(73) Patentinhaber: **Hochschule Karlsruhe Technik und Wirtschaft**
**76133 Karlsruhe (DE)**

(72) Erfinder:
• **Kohler, Heinz**
  **77815 Bühl (DE)**
• **Ojha, Binayak**
  **76185 Karlsruhe (DE)**
• **Knoblauch, Jens**
  **79286 Glottertal (DE)**

(74) Vertreter: **Ege Lee & Partner Patentanwälte PartGmbB Walter-Gropius-Straße 15 80807 München (DE)**

(56) Entgegenhaltungen:
**DE-A1- 19 832 411     US-A1- 2010 043 528**

• **AHMADI MOZHGAN ET AL: "Development of a PID based on-line tar measurement method - Proof of concept", FUEL, IPC SCIENCE AND TECHNOLOGY PRESS, GUILDFORD, GB, Bd. 113, 7. Juni 2013 (2013-06-07), Seiten 113-121, XP028698602, ISSN: 0016-2361, DOI: 10.1016/J.FUEL.2013.05.075**
• **DEFOORT F ET AL: "A promising new on-line method of tar quantification by mass spectrometry during steam gasification of biomass", BIOMASS AND BIOENERGY, PERGAMON, AMSTERDAM, NL, Bd. 65, 19. April 2014 (2014-04-19), Seiten 64-71, XP029026787, ISSN: 0961-9534, DOI: 10.1016/J.BIOMBIOE.2014.04.001**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Erfassung eines Teergehalts in einem oxidierbare Komponenten enthaltenden Messgasgemisch und ein Messsystem zur Durchführung des Verfahrens.

Gattungsgemäße Messgasgemische treten bei der Umwandlung von biogenen Ausgangsstoffen, beispielsweise in Schwelgasen einer Holzvergasung oder dergleichen auf und werden als Synthesegas zur Herstellung von organischen Verbindungen, als Betriebsstoff von Gasmotoren oder dergleichen eingesetzt. Hauptbestandteile des Messgasgemisches sind dabei Methan, Kohlenmonoxid und weitere oxidierbare Komponenten. Nachteilig bei derartigen Vergasungsvorgängen ist die Bildung von Teer. Die Zusammensetzung dieses Teers ist abhängig von dem zugrunde liegenden biogenen Ausgangsstoff und den Verfahrensparametern des Verfahrens der Vergasung des biogenen Ausgangsstoffs. Es ist daher vorteilhaft, den Teergehalt in einfacher Weise als Summenparameter zu ermitteln. Einfache Messsysteme zur Ermittlung des Teergehalts beispielsweise zur Regelung einer Anlage zur Erzeugung des Messgasgemisches mit einem minimierten Teergehalt stehen für einen Einsatz vor Ort nicht zur Verfügung.

Beispielsweise ist aus der Druckschrift MOERSCH, O.; SPLIETHOFF, H.; HEIN, K. R. G. Tar quantification with a new online analyzing method. Biomass and Bioenergy, 2000, 18. Jg., Nr. 1, S. 79-86 ein Verfahren und eine Vorrichtung bekannt, bei dem ein Teergehalt anhand von Messsignalen eines Flammenionisationsdetektors (FID) ermittelt wird. Hierbei werden organische Verbindungen anhand einer Leitfähigkeit einer Knallgasflamme ermittelt. Der Teergehalt wird bestimmt, indem Signale des FID mit und ohne Abreicherung von Teer miteinander verglichen werden. Neben der aufwendigen Ausbildung und Art dieser Messmethode werden mit diesem Nachweis die organischen Komponenten samt Teer direkt nachgewiesen, so dass ein geringer Teergehalt vor dem Hintergrund eines großen Gehalts an anderen oxidierbaren Komponenten beispielsweise in Synthesegas ermittelt werden muss, so dass neben dem Hintergrundsignal der oxidierbaren Komponenten ein geringes Messsignal des Teergehalts ausgewertet werden muss.

Aufgabe der Erfindung ist die Weiterbildung eines Verfahrens und eines Messsystems zur Ermittlung des Teergehalts.

Die Aufgabe wird durch den Gegenstand des Verfahrens gemäß Anspruchs 1 gelöst. Weiterhin wird die Aufgabe durch die Vorrichtung des Anspruchs 11 gelöst. Die von den Ansprüchen 1 und 11 abhängigen Ansprüche geben vorteilhafte Ausführungsformen der Gegenstände der Ansprüche 1 und 11 wieder.

Das vorgeschlagene Verfahren dient der Erfassung eines Teergehalts in einem oxidierbare Komponenten enthaltenden Messgasgemisch. Beispielsweise kann das Verfahren zur Steuerung einer Vergasungsanlage von biogenen Ausgangsstoffen, beispielsweise eines Holzvergasers eingesetzt werden, um den Teergehalt zu minimieren, indem entsprechende Führungsgrößen der Vergasung abhängig vom ermittelten Teergehalt adaptiert werden. Eine Erfassung des in der Regel sowohl stöchiometrisch als auch bezüglich dessen chemischen Zusammensetzung heterogenen Teers erfolgt in bevorzugter Weise als Summenparameter. Die Erfassung des Teergehalts erfolgt mittels eines Sauerstoffsensors, beispielsweise eines elektrochemischen Sauerstoffsensors beispielsweise nach dem amperometrischen und/oder potentiometrischen Messprinzip. In bevorzugter Weise ist als Sauerstoffsensor eine Lambdasonde vorgesehen. Der Sauerstoffsensor ermittelt nach Zufuhr eines vorgegebenen Sauerstoffgehalts in das Messgasgemisch bei anwesenden oxidierbaren Komponenten beispielsweise Methan, Kohlenmonoxid und dergleichen sowie dem zu erfassenden Teergehalt nach Umsetzung der oxidierbaren Komponenten mit dem zugeführten Sauerstoff verbleibende Restgehalte an Sauerstoff, Dies bedeutet, dass der Gehalt an Teer indirekt erfasst wird, indem der Verbrauch von Sauerstoff durch die Summe aller oxidierbaren Komponenten abzüglich eines Verbrauchs an Sauerstoff ohne den Teergehalt ermittelt wird. Auf diese Weise kann der Vergleich von Restgehalten des Sauerstoffs mit und ohne Teer gegenüber einer direkten Bestimmung der organischen Zusammensetzung mit und ohne Teer gemäß dem Stand der Technik verbessert durchgeführt werden, da die Restgehalte an Sauerstoff von den verbleibenden oxidierbaren Komponenten hintergrundbereinigt ermittelt und dem Teergehalt zugeordnet werden können. Beispielsweise können die Restgehalte an Sauerstoff einer Umsetzung der oxidierbaren Komponenten des teerhaltigen Messgasgemischs mit Restgehalten an Sauerstoff einer Umsetzung der oxidierbaren Komponenten eines Messgasgemischs mit entferntem Teergehalt verglichen und daraus der Einfluss der oxidierbaren Komponenten ohne Teergehalt eliminiert werden. Mittels Kalibration kann von dem ermittelten Restgehalt an Sauerstoff auf den Teergehalt geschlossen werden. Hierzu kann beispielsweise vorab eine Kalibrierkurve des Teergehalts über ein von dem Sauerstoffsensor erzeugtes Messsignal erstellt und das aktuell erfasst Messsignal mit der Kalibrierkurve verglichen werden. Der Teergehalt ist hierbei beispielsweise äquivalent zu einem auf einen Sauerstoffpartialdruck rückführbaren Messsignal wie einem Messstrom, einer Messspannung, einem Pumpstrom der Lambdasonde oder dergleichen.

Bevorzugt wird eine sogenannte Breitband-Lambdasonde eingesetzt, deren Aufbau und Funktionsweise beispielsweise aus der Druckschrift DE 10 2013 204 463 A1 bekannt ist. Eine derartige Lambdasonde mit Breitbandcharakteristik ermöglicht eine Erfassung von Sauerstoffgehalten, beispielsweise der Restgehalte an Sauerstoff über zumindest eine Größenordnung, bevorzugt mehreren Größenordnungen einer Sauerstoffkonzentration, auch bei Sauerstoffgehalten außerhalb des Bereiches stöchiometrischer Gemische mit einem Lambdawert gleich eins. Alternativ oder zusätzlich kann bei genügend genauer Einstellung eines Gehalts an Sauerstoff auf einen Lambdawert gleich eins und entsprechend geringen Änderungen des Gehalts durch die oxidierbaren Komponenten samt Teer eine schnelle und bevorzugt innerhalb eines Lambdawerts von eins arbeitende Lambdasonde mit Schmalbandcharakteristik vorgesehen werden. An derart oder ähnlich ausgebildeten Lambdasonden erfolgt eine katalytische Umsetzung der oxidierbaren Komponenten einschließlich des gegebenenfalls vorliegenden Teergehalts unter Sauerstoffverbrauch, so dass der verbleibende Restgehalt an Sauerstoff verglichen mit dem Gehalt an Sauerstoff in einer Vergleichsgasmischung, beispielsweise Umgebungsluft abhängig von den Gehalten an oxidierbaren Komponenten ist. Mittels Kondensation des Teergehalts kann ein Restgehalt an Sauerstoff mit und ohne den Teergehalt ermittelt werden und daraus auf den Summenparameter des Teergehalts geschlossen werden.

Das vorgeschlagene Verfahren enthält zumindest die Verfahrensschritte:

- Entnahme zumindest eines Teilstroms eines Messgasgemisches,
- Zudosierung eines vorgegebenen Gehalts an Sauerstoff zu dem zumindest einen Teilstrom,
- Änderung des Gehalts an Sauerstoff durch Umsetzung von in dem zumindest einen Teilstrom vorhandenen oxidierbaren Komponenten zu Restgehalten an Sauerstoff,
- Ermittlung eines von einer mittels einer Abreicherungseinrichtung durchgeführten Abreicherung des Teergehalts abhängigen ersten Restgehalts an Sauerstoff mittels zumindest eines Sauerstoffsensors,
- Ermittlung eines zweiten Restgehalts an Sauerstoff mittels zumindest eines Sauerstoffsensors ohne Abreicherung des Teergehalts,
- Ermittlung eines Summenparamters des Teergehalts aus einem Vergleich der beiden Restgehalte an Sauerstoff.

Gemäß einer vorteilhaften Durchführung des vorgeschlagenen Verfahrens wird dem Teilstrom des Synthesegases in einem Regelungsprozess eine bestimmte Menge Sauerstoff, beispielsweise in Form von synthetischer Luft zudosiert, so dass beispielsweise ein stöchiometrisches Gemisch, welches definitionsgemäß einem Lambdawert von eins entspricht, entsteht. Anschließend wird nach Oxidation der oxidierbaren Komponenten der Restgehalt an Sauerstoff ROC(M) gemessen. Anschließend wird bei unveränderter Zugabe von Sauerstoff der Teilstrom über die Abreicherungseinrichtung geführt und nach Oxidation der verbleibenden oxidierbaren Komponenten der Restgehalt ROC(R) gemessen. Der Summenparameter des Teergehalts wird mittels der nachfolgenden Gleichung Gl. 1 ermittelt:

$$SP(Teer) \propto ROC(R) - ROC(M) \qquad Gl.\ 1$$

Demnach ist der Summenparameter SP(Teer) des Teergehalts proportional zur Differenz aus dem Restgehalt an Sauerstoff ohne Teergehalt ROC(R) und dem Restgehalt an Sauerstoff mit Teergehalt ROC(M).

Die Restgehalte an Sauerstsoff können dabei in zwei identischen Messaufbauten mit Ausnahme einer wirksamen Abreicherungseinrichtung in einem der Messaufbauten ermittelt werden. Hierbei wird in beide Messaufbauten derselbe Gehalt an Sauerstoff zugegeben.

In einer alternativen Ausführungsform kann ein einziger Messaufbau vorgesehen werden, indem die Restgehalte an Sauerstoff in diesem Messaufbau zeitlich getaktet mittels eines über die Abreicherungseinrichtung geführten Teilstroms und eines an der Abreicherungseinrichtung vorbei geführten Teilstroms ermittelt werden. Ist dabei die zeitliche Taktung schneller als ein Wechsel der Zusammensetzung des Messgasgemisches, kann eine ausreichend genaue Trennung und Auswertung der Restgehalte ROC(M), ROC(R) gemäß Gleichung Gl. 1 zwischen den einzelnen Zeittakten erfolgen und damit ein quasi kontinuierliches Messsignal ausgegeben werden.

Der Teilstrom beziehungsweise die Teilströme mit der aus dem Messgasgemisch und dem zugeführten Gehalt an Sauerstoff gebildeten Zusammensetzung können bei einer Verwendung einer Lambdasonde als Sauerstoffsensor direkt an der Lambdasonde vorbeigeführt werden, so dass die umfassende Umsetzung der mittels Diffusion an die Lambdasonde herangeführten oxidierbaren Komponenten an der Lambdasonde erfolgt. Hierbei wird von dem Teilstrom ein Arbeitsbereich der Lambdasonde abgetrennt. An den Elektroden der Lambdasonde werden dabei die im Arbeitsbereich befindlichen oxidierbaren Komponenten umgesetzt und der Sauerstoffgehalt erfasst. Durch das im Verhältnis zu dem Teilstrom vernachlässigbare Volumen des Arbeitsbereichs bleibt die Zusammensetzung des Teilstroms praktisch unverändert, so dass der Messung der Lambdasonde im Teilstrom nachfolgende Messungen im Wesentlichen unverfälscht bleiben.

In einer alternativen Ausführung des Verfahrens kann dem Sauerstoffsensor, der hier ebenfalls eine Lambdasonde sein kann, ein Katalysator zur Oxidation der oxidierbaren Komponenten vorgeschaltet werden. An dem Katalysator werden die oxidierbaren Komponenten einschließlich einem gegebenenfalls vorhandenen Teergehalt unter stöchiometrischem Sauerstoffverbrauch oxidiert, so dass ein Unterschied des Restgehalts an Sauerstoff abhängig vom Teergehalt mit beziehungsweise ohne Abreicherung erhalten bleibt und entsprechend an dem Sauerstoffsensor erfasst wird. Wird das Verfahren ohne Katalysatoren durchgeführt, kann die Lambdasonde zur Vermeidung eines Zündens des mit Sauerstoff angereicherten und gegebenenfalls zündfähigen Messgasgemisches hinter einer Diffusionsmembran angebracht werden.

Gemäß einer weiteren vorteilhaften Ausführungsform des vorgeschlagenen Verfahrens kann der zumindest eine Teilstrom des aus dem Synthesegas entnommenen Mischgasgemisches mittels eines weiteren Sauerstoffsensors, beispielsweise einer Lambdasonde mit Schmalbandcharakteristik, beispielsweise einer PT/YSZ-Lambdasonde eingestellt und bestimmt werden. Der Teilstrom des Messgasgemisches wird in einem Regelungsprozess mit Sauerstoff, beispielsweise synthetischer Luft angereichert. Beispielsweise wird der Lambdawert gleich eins eingestellt. Wechselweise wird bei gleicher Zudosierung von Sauerstoff der Teilstrom durch eine Abreicherungseinrichtung, beispielsweise eine Kühlfalle oder dergleichen geführt. In der Abreicherungseinrichtung wird der Teergehalt abgereichert, beispielsweise kondensiert, so dass das mittels der Lambdasonde eingestellte stöchiometrische Verhältnis durch den fehlenden Teergehalt gestört wird. Die anschließende Ermittlung des Restgehalts an Sauerstoff erfolgt unter vollständiger Umsetzung der verbliebenen oxidierbaren Komponenten an dem als Lambdasonde ausgebildeten, messenden Sauerstoffsensor. Durch den in der Abreicherungseinrichtung abgereicherten Teergehalt wird das mittels der Dosiereinrichtung eingestellte Verhältnis zwischen den oxidierbaren Komponenten und Sauerstoff zugunsten des Restgehalts an Sauerstoff entsprechend dem stöchiometrischen Anteil des zurückgehaltenen Teers verschoben, so dass der Restgehalt an Sauerstoff abhängig vom in dem vor der Abreicherungseinrichtung vorliegenden Teergehalt höher ist und von der Lambdasonde bei Umsetzung der verbliebenen oxidierbaren Komponenten ein von dem Teergehalt abhängiger Sauerstoffpartialdruck beziehungsweise Pumpstrom ermittelt wird.

Gemäß einer weiteren vorteilhaften Ausführungsform des vorgeschlagenen Verfahrens können Teilströme mittels wechselweiser Taktung des Messgasstroms erzeugt werden. Hierbei kann ein erster Teilstrom an der Abreicherungseinrichtung ohne Abreicherung des Teergehalts vorbeigeführt und ein zweiter Teilstrom durch die Abreicherungseinrichtung unter Abreicherung des Teergehalts geführt werden. Dem ersten Teilstrom ohne abgereicherten Teer wird mittels eines ersten Sauerstoffsensors geregelt ein vorgegebener Sauerstoffgehalt zugeführt und in einem zweiten, als Lambdasonde ausgeführten Sauerstoffsensor wird unter vollständiger Umsetzung der oxidierbaren Komponenten im Arbeitsbereich der Lambdasonde der Restgehalt an Sauerstoff ermittelt. Anschließend wird dem mit in der Abreicherungseinrichtung abgereicherten Teergehalt zweiten Teilstrom derselbe Sauerstoffgehalt zugeführt und in dem zweiten Sauerstoffsensor unter Umsetzung der verbliebenen, im Arbeitsbereich der Lambdasonde befindlichen oxidierbaren Komponenten der Restgehalt an Sauerstoff bestimmt. Durch Vergleich der beiden hierdurch ermittelten Restgehalte an Sauerstoff wird der Teergehalt ermittelt.

Zur Regelung des Gehalts an zugeführtem Sauerstoff kann ein separater Sauerstoffsensor eingesetzt werden. Alternativ kann zur Vereinfachung des Messaufbaus als erster Sauerstoffsensor zur Regelung des Sauerstoffgehalts des zumindest einen Teilstroms und als zweiter Sauerstoffsensor zur Bestimmung der Restgehalte nach Umsetzung der oxidierbaren Komponenten derselbe Sauerstoffsensor eingesetzt werden. Im Falle einer vorgesehenen Umsetzung der oxidierbaren Komponenten ohne Katalysator kann der gemeinsame Sauerstoffsensor als Lambdasonde ausgebildet sein.

Insbesondere bei einer Verwendung einer Lambdasonde mit Schmalbandcharakteristik kann zumindest der den Sauerstoffgehalt regelnde Sauerstoffsensor in dem zumindest einen Teilstrom einen Sauerstoffgehalt in dem zumindest einen Teilstrom auf einen Lambdawert wie Luftzahl gleich eins einstellen.

Die Aufgabe wird zudem durch ein Messsystem zur Durchführung des Verfahrens gemäß den vorgehend beschriebenen Merkmalen gelöst. Das Messsystem enthält einen Messaufbau mit einer Pumpe und einer Dosiereinrichtung, beispielsweise einem Massendurchflussregler, Volumendurchflussregler oder dergleichen für ein Reaktionsgas mit vorgegebenem Sauerstoffgehalt, beispielsweise Umgebungsluft oder synthetische Luft. Das Volumen beziehungsweise der Volumen- oder Massenstrom der Dosiereinrichtung kann erfasst und der Teilstrom bei durch die Pumpe vorgegebenem Gesamtgasstrom bestimmt werden.

Zur Entfernung oder Abreicherung des Teergehalts und gegebenenfalls des die Messung des Restgehalts an Sauerstoff störenden Wassers beziehungsweise Wasserdampfs ist eine Abreicherungseinrichtung, beispielsweise Kühlfalle, die beispielsweise mittels eines Peltierelements betrieben sein kann, oder dergleichen vorgesehen. Zur Erfassung und Ermittlung des abhängig von der Abreicherung des Gehalts an Teer verbleibenden Restgehalts an Sauerstoff ist ein Sauerstoffsensor, beispielsweise eine Lambdasonde, beispielsweise eine Breitband-Lambdasonde oder eine Sprungsonde vorgesehen. Zur Erfassung und Auswertung der Signale des Sauerstoffsensors ist zur Steuerung der Dosiereinrichtung eine Auswerteeinrichtung vorgesehen.

Das vorgeschlagene Messsystem enthält in einer ersten Ausprägung einen zweiten Messaufbau, der bis auf eine fehlende Abreicherungseinrichtung identisch mit dem genannten Messaufbau und diesem parallel geschaltet ist. Hierbei wird in dem Messaufbau mit der Abreicherungseinrichtung der Restgehalt an Sauerstoff ohne den Einfluss des Teergehalts an der Lambdasonde erfasst und an dem zweiten Messaufbau der Restgehalt an Sauerstoff mit dem Einfluss des Teergehalts erfasst. In einer alternativen Ausprägung dieses Messsystems ist nur ein einziger Messaufbau vorgesehen, bei dem mittels einer entsprechenden Umschaltungseinrichtung der Teilstrom zeitlich getaktet über die Abreicherungseinrichtung und im Bypass vorgesehen ist. Die Umschalteinrichtung kann beispielsweise als Dreiwegeventil ausgebildet sein. Die Umschalteinrichtung kann von der Auswerteeinrichtung gesteuert sein. Die Umschaltung des Teilstroms kann vor der Abreicherungseinrichtung erfolgen. Hierbei wird mittels der Umschalteinrichtung ein originärer Teilstrom in einen Teilstrom über die Abreicherungseinrichtung und einen Teilstrom im Bypass verzweigt, wobei diese anschließend wiedervereinigt werden. Alternativ kann aus dem Abgas des Vergasers ein erster Teilstrom abgezweigt und über eine Abreicherungseinrichtung und ein zweiter Teilstrom im Bypass auf die Umschalteinrichtung geführt sein, wobei die Umschalteinrichtung beide Teilströme in einen gemeinsamen Teilstrom des Messaufbaus zeitlich getaktet schaltet.

In der Auswerteeinrichtung wird aus den beiden Restgehalten entsprechend GI. 1 der Summenparameter für den Teergehalt bestimmt. Zur vollständigen Entfernung der oxidierbaren Komponenten einschließlich des Teergehalts kann eine Umsetzungseinrichtung vorgesehen sein. Beispielsweise kann vor den die Restgehalte an Sauerstoff erfassenden Sauerstoffsensor ein die Umsetzung beschleunigender Katalysator, beispielsweise ein PT-Katalysator geschaltet sein. Alternativ oder zusätzlich kann bei einem als Lambdasonde ausgebildeten Sauerstoffsensor die Umsetzungseinrichtung in der Lambdasonde vorgesehen sein, indem diese die oxidierbaren Komponenten umsetzt.

Gemäß einer alternativen Ausführungsform des Messsystems kann der vorgegebene Sauerstoffgehalt mittels einer zusätzlichen, der Dosiereinrichtung nachgeschalteten und der Abreicherungseinrichtung vorgeschalteten, zusätzlichen Lambdasonde geregelt einstellbar vorgesehen sein.

Die Erfindung wird anhand der in den Figuren 1 bis 4 dargestellten Ausführungsbeispiele näher erläutert. Diese zeigen:

Figur 1 ein Messsystem zur Ermittlung eines Summenparameters eines Teergehalts in schematischer Darstellung,
Figur 2 ein gegenüber dem Messsystem der Figur 1 abgeändertes Messsystem in schematischer Darstellung,
Figur 3 ein gegenüber den Messsystemen der Figuren 1 und 2 mit einem alternativen Messprinzip arbeitendes Messsystem in schematischer Darstellung
und
Figur 4 ein gegenüber den Messsystemen 1 bis 3 mit einem alternativen

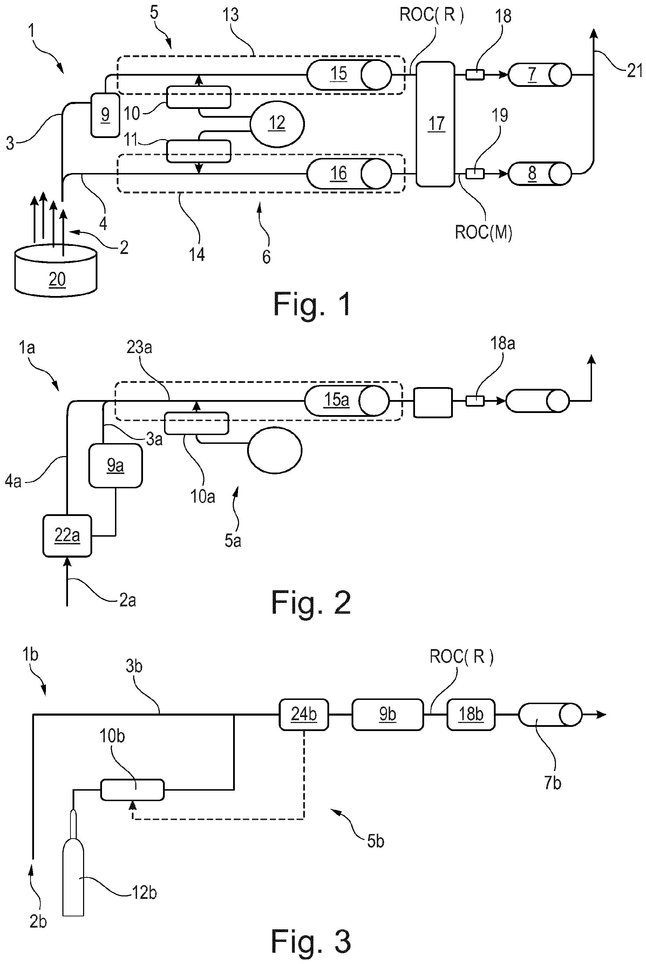

Messprinzip arbeitendes Messsystem in schematischer Darstellung. Die Figur 1 zeigt das Messsystem 1 zur Erfassung eines Summenparameters eines Teergehalts in schematischer Darstellung. In dem Vergaser 20 wird aus biogenem Ausgangsstoff das Messgasgemisch 2, beispielsweise Synthesegas mit verschiedenen oxidierbaren Komponenten, beispielsweise Methan, Kohlenmonoxid und dergleichen freigesetzt. In dem Messgasgemisch 2 sind weiterhin unerwünschte Komponenten, beispielsweise der zu erfassende Teergehalt enthalten.

Aus dem Messgasgemisch 2 werden die Teilströme 3, 4 abgezweigt und den beiden Messaufbauten 5, 6 zugeführt. Die Teilströme 3, 4 werden jeweils mittels der Pumpen 7, 8 durch die Messaufbauten 5, 6 geleitet.

Der Messaufbau 5 weist die Abreicherungseinrichtung 9, beispielsweise eine Kühlfalle auf, in der der wesentliche Teergehalt mittels Kondensation zurückgehalten wird. Im Übrigen sind die Messaufbauten 5, 6 identisch ausgebildet. Zur Erhöhung deren Symmetrie kann an der Stelle der Abreicherungseinrichtung 9 ein ähnlicher Behälter ohne Kühlung vorgesehen sein.

Zu den Teilströmen 3, 4 wird jeweils mittels der Dosiereinrichtungen 10, 11 jeweils der identische Gehalt an Sauerstoff aus dem Druckbehälter 12 beispielsweise mit synthetischer Luft oder vorverdichteter Umgebungsluft dosiert und damit ein stöchiometrischer oder überstöchiometrischer Gehalt an Sauerstoff eingestellt. Mittels der Heizungen 13, 14 werden die Teilströme auf eine Reaktionstemperatur, beispielsweise größer 300°C, der oxidierbaren Komponenten mit dem Sauerstoff an den Katalysatoren 15, 16 gebracht. Alternativ können die Heizungen 13, 14 lediglich einer Vermeidung von Kondensationen dienen und die Katalysatoren 15, 16 separat auf die notwenige Betriebstemperatur beheizt sein. An den Katalysatoren 15, 16 erfolgt eine im Wesentlichen vollständige Umsetzung der oxidierbaren Komponenten unter stöchiometrischem Verbrauch von Sauerstoff. Dabei wird in dem Messaufbau 5 infolge des in der Abreicherungseinrichtung 9 zurückgehaltenen Teergehalts weniger Sauerstoff umgesetzt als in dem Messaufbau 6.

Die Teilströme 3, 4 mit den Restgehalten ROC(M), ROC(R) werden in dem Kühler 17 abgekühlt und den Sauerstoffsensoren 18, 19, beispielsweise Lambdasonden wie Sprungsonden oder Breitband-Lambdasonden zugeführt, beziehungsweise an diesen vorbeigeführt. Die Ausgangsgrößen der Sauerstoffsensoren werden von einer nicht dargestellten Auswerteeinrichtung erfasst und beispielsweise gemäß GI. 1 miteinander verglichen, so dass einer Differenz der erfassten Messgrößen mittels Kalibration ein Summenparameter des Teergehalts zugeordnet werden kann.

Zur Vereinfachung des Verfahrens können die Katalysatoren 15, 16 und die Heizungen 13, 14 sowie der Kühler 17 weggelassen werden. Die stöchiometrische Umsetzung der oxidierbaren Komponenten und gegebenenfalls des Teergehalts erfolgt dabei im Diffusionsbereich der als Lambdasonden ausgebildeten Sauerstoffsensoren 18, 19. Die Sauerstoffsensoren 18, 19 sind hierbei gegenüber den Teilströmen 3, 4 über Diffusion verbunden, jedoch zur Vermeidung von Zündungen explosionsgeschützt isoliert.

Der Gasauslass 21 führt das Messgas beispielsweise in den Synthesegasstrom zurück.

Die Figur 2 zeigt das gegenüber dem Messsystem 1 der Figur 1 vereinfachte Messsystem 1a in schematischer Darstellung. Das Messsystem 1a verfügt über lediglich einen einzigen Messaufbau 5a, in dem das Messgasgemisch 2a von dem nicht dargestellten Vergaser auf das Dreiwegeventil 22a geleitet wird. Das Dreiwegeventil 22a leitet das Messgasgemisch 2a zeitlich getaktet in zwei Teilströme 3a, 4a. Der Teilstrom 3a führt das Messgasgemisch 2a über die Abreicherungseinrichtung 9a, der Teilstrom 4a führt das Messgas im Bypass an der Abreicherungseinrichtung 9a vorbei. Beide Teilströme 3a, 4a werden anschließend in dem Teilstrom 23a vereinigt.

Mittels der Dosiereinrichtung 10a wird ein Gehalt an Sauerstoff, der zu einer Luftzahl größer eins in dem Teilstrom 23a führt, zudosiert.

Der weitere Verlauf des Verfahrens erfolgt analog zu dem Verfahren des Messsystems 1 wobei in Abhängigkeit der zeitlichen Taktung an dem Katalysator 15a wechselweise die oxidierbaren Komponenten mit und ohne Teergehalt stöchiometrisch umgesetzt werden und der Sauerstoffsensor 18a ein Wechselsignal aus Restgehalten an Sauerstoff ROC(M), ROC(R) erzeugt, welches entsprechend Gl. 1 ausgewertet wird. Die Taktrate des Dreiwegeventils 22a erfolgt in Abstimmung mit der zeitlichen Abhängigkeit einer Änderung der Gehalte an oxidierbaren Komponenten und des Teergehalts im Messgasgemisch 2a.

Die Messsysteme 1, 1a der Figuren 1 und 2 können jeweils ohne Katalysatoren ausgebildet sein, wobei als Sauerstoffsensoren 18, 18a, 19 Lambdasonden eingesetzt werden, die eine entsprechende Umsetzung der oxidierbaren Komponenten mit und ohne Teer an ihren aus Platin gebildeten Elektroden vorsehen und damit entsprechende Restgehalte an Sauerstoff erfassen.

Die Figur 3 zeigt das mit einem gegenüber dem Messprinzip der Messsysteme 1, 1a der Figuren 1 und 2 abgeänderten Messprinzip arbeitende Messsystem 1b in schematischer Darstellung. Das Messgasgemisch 2b wird in den Teilstrom 3b eingeleitet und mittels der Dosiereinrichtung 10b mit einem vorgebbaren Gehalt an Sauerstoff, beispielsweise wie hier aus der Druckluftflasche 12b versehen. Die Pumpe 7b führt den Teilstrom 3b durch den Messaufbau 5b, wobei eine durch die Dosierung von Reaktionsgas mit Sauerstoff mittels der Dosiereinrichtung 10b veränderte Konzentration der oxidierbaren Komponenten und des Teergehalts des ursprünglichen Teilstroms 3b mittels der Auswertung des Volumenstroms der Dosiereinrichtung 10b auf die ursprüngliche Konzentration dieser rückgeführt wird.

Im Unterschied zu den Messsystemen 1, 1a, bei denen jeweils ein exakt stöchiometrischer oder überstöchiometrischer konstanter Gehalt an Sauerstoff dosiert wird, wird die Dosiereinrichtung 10b mittels des in dem Teilstrom 3b messenden Sauerstoffsensors 24b, bevorzugt einer beispielsweise als Sprungsonde ausgebildeten Lambdasonde geregelt. Der Sauerstoffsensor 24b regelt den Gehalt an Sauerstoff auf einen vorgebbaren stöchiometrischen oder überstöchiometrischen Wert, so dass Sauerstoff einerseits und die oxidierbaren Komponenten und der Teergehalt andererseits entsprechende Reaktionsbedingungen aufweisen. Der auf diese Weise eingestellte Teilstrom 3b wird anschließend in die Abreicherungseinrichtung 9b überführt, in der der Teergehalt kondensiert. Der verbleibende Teilstrom 3b steht gegebenenfalls explosionsgeschützt über Diffusion mit dem Sauerstoffsensor 18b, der als Lambdasonde beispielsweise als Breitband-Lambdasonde ausgebildet ist, in Verbindung. Der als Lambdasonde ausgebildete Sauerstoffsensor 18b misst den Restgehalt an Sauerstoff ROC(R) bei Umsetzung der verbleibenden oxidierbaren Komponenten ohne den Teergehalt, wobei eine dem Teergehalt entsprechende Differenz des dosierten Gehalts an Sauerstoff mit Teer und des von dem Sauerstoffsensor 18b nach Abreicherung gemessenen Restgehalts resultiert. Der Summenparameter des Teergehalts kann über Kalibration dieser Differenz quantifiziert werden.

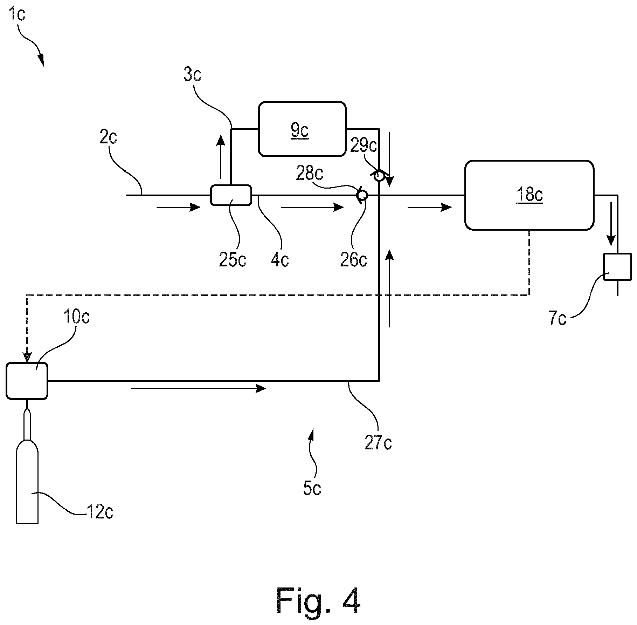

Die Figur 4 zeigt das Messsystem 1 c mit dem einzigen Messaufbau 5c in schematischer Darstellung. Mittels der Pumpe 7c wird beispielsweise aus einem nicht dargestellten Vergaser das Messgasgemisch 2c, beispielsweise Synthesegas mit oxidierbaren Komponenten zur Verbrennung oder Weiterverarbeitung mit einer Teerbelastung angesaugt und wechselweise, beispielsweise zeitlich getaktet von dem Ventil 25c, beispielsweise einem Dreiwegeventil in die Teilströme 3c, 4c geteilt. In dem Teilstrom 3c wird der vorhandene Teergehalt mittels der Abreicherungseinrichtung 9c entfernt. Der Teilstrom 4c verbleibt ohne Abreicherung des Teergehalts. An der Verbindungsstelle 26c werden die Leitungen der Teilströme 3c, 4c und die Leitung 27c zur Versorgung mit sauerstoffhaltigem Reaktionsgas, beispielsweise synthetischer Luft oder reiner wie gereinigter Umgebungsluft zusammengeführt. Ein vorgegebener Gehalt an Sauerstoff, beispielsweise zur Einstellung des Lambdawerts wie Luftzahl gleich eins erfolgt in dem gezeigten Ausführungsbeispiel mittels des Sauerstoff enthaltenden Druckbehälters 12c und der Dosiereinrichtung 10c. Die Dosiereinrichtung 10c wird gegebenenfalls unter Zwischenschaltung eines Mess- und Auswertesystems von dem als Lambdasonde ausgebildeten Sauerstoffsensor 18c gesteuert. Dem Teilstrom 4c wird dabei so viel Sauerstoff zugeführt, dass der Gehalt an Sauerstoff in dem Teilstrom 4c beispielsweise dem Lambdawert gleich eins entspricht. Dieser durch die Dosiereinrichtung 10c eingestellte Gehalt an Sauerstoff wird beibehalten, wenn das Ventil 25c auf den Teilstrom 3c umschaltet.

Je nach Schaltung des Ventils 25c wird dem Sauerstoffsensor 18c zuerst der Teilstrom 4c mit Teer und anschließend der Teilstrom 3c mit abgereichertem Teer bei konstant eingestelltem Gehalt an Sauerstoff zugeführt. Die Rückschlagventile 28c, 29c vermeiden dabei einen Rückfluss der jeweiligen Teilströme 3c, 4c in die Leitung des anderen Teilstroms. Fließt der Teilstrom 3c an dem Sauerstoffsensor 18c vorbei, wird an diesem unter vollständiger Oxidation der oxidierbaren Komponenten ausschließlich der um den Sauerstoff zur Oxidation der oxidierbaren Komponenten ohne Teer verringerte Restgehalt ROC(R) erfasst. Fließt der Teilstrom 4c an dem Sauerstoffsensor 18c vorbei, wird an diesem unter vollständiger Oxidation der oxidierbaren Komponenten der um den Sauerstoff zur Oxidation der oxidierbaren Komponenten und des Teergehalts verringerte Restgehalt ROC(M) erfasst. Der Vergleich der Restgehalte ROC(R) und ROC(M) liefert gemäß der Gl. 1 des vorhergehenden Textes den Summenparameter SP(Teer) für den Teergehalt.

Es versteht sich, dass der Sauerstoffsensor 18c als Lambdasonde mit Schmalbandcharakteristik und eine Lambdasonde mit Breitbandcharakteristik oder aus einer Kombination dieser ausgebildet sein kann.

**Bezugszeichenliste**

| | |
|------|------|
| 1 | Messsystem |
| 1 a | Messsystem |
| 1 b | Messsystem |
| 1c | Messsystem |
| 2 | Messgasgemisch |
| 2a | Messgasgemisch |
| 2b | Messgasgemisch |
| 2c | Messgasgemisch |
| 3 | Teilstrom |
| 3a | Teilstrom |
| 3b | Teilstrom |
| 3c | Teilstrom |
| 4 | Teilstrom |
| 4a | Teilstrom |
| 4c | Teilstrom |
| 5 | Messaufbau |
| 5a | Messaufbau |
| 5b | Messaufbau |
| 5c | Messaufbau |
| 6 | Messaufbau |
| 7 | Pumpe |
| 7b | Pumpe |
| 7c | Pumpe |
| 8 | Pumpe |
| 9 | Abreicherungseinrichtung |
| 9a | Abreicherungseinrichtung |
| 9b | Abreicherungseinrichtung |
| 9c | Abreicherungseinrichtung |
| 10 | Dosiereinrichtung |
| 10a | Dosiereinrichtung |
| 10b | Dosiereinrichtung |
| 10c | Dosiereinrichtung |
| 11 | Dosiereinrichtung |
| 12 | Druckbehälter |
| 12b | Druckluftflasche |
| 12c | Druckbehälter |
| 13 | Heizung |
| 14 | Heizung |
| 15 | Katalysator |
| 15a | Katalysator |
| 16 | Katalysator |
| 17 | Kühler |
| 18 | Sauerstoffsensor |
| 18a | Sauerstoffsensor |
| 18b | Sauerstoffsensor |
| 18c | Sauerstoffsensor |
| 19 | Sauerstoffsensor |
| 20 | Vergaser |
| 21 | Gasauslass |
| 22a | Dreiwegeventil |

| 23a | Teilstrom |
|---|---|
| 24b | Sauerstoffsensor |
| 25c | Ventil |
| 26c | Verbindungsstelle |
| 27c | Leitung |
| 28c | Rückschlagventil |
| 29c | Rückschlagventil |
| ROC(M) | Restgehalt Sauerstoff |
| ROC(R) | Restgehalt Sauerstoff |

**Patentansprüche**

1. Verfahren zur Erfassung eines Teergehalts in einem oxidierbare Komponenten enthaltenden Messgasgemisch (2, 2a, 2b, 2c) zumindest enthaltend folgende Verfahrensschritte:

   - Entnahme zumindest eines Teilstroms (3, 3a, 3b, 3c, 4, 4c, 23a) des Messgasgemisches (2, 2a, 2b, 2c),
   - Zudosierung eines vorgegebenen Gehalts an Sauerstoff zu dem zumindest einen Teilstrom (3, 3c, 4, 4c, 23a),
   - Änderung des Gehalts an Sauerstoff durch Umsetzung einer vorgegebenen Menge von in dem zumindest einen Teilstrom (3, 3a, 3b, 3c, 4, 4c, 23a) vorhandenen oxidierbaren Komponenten zu Restgehalten an Sauerstoff ROC(R), ROC(M)),
   - Ermittlung eines von einer mittels einer Abreicherungseinrichtung (9, 9a, 9b) durchgeführten Abreicherung des Teergehalts abhängigen ersten Restgehalts an Sauerstoff (ROC(R)) mittels zumindest eines Sauerstoffsensors (18, 18a, 18b, 18c, 19),
   - Ermittlung eines zweiten Restgehalts an Sauerstoff (ROC(M)) mittels zumindest eines Sauerstoffsensors (18, 18a, 18b, 18c, 19) ohne Abreicherung des Teergehalts,
   - Ermittlung eines Summenparameters des Teergehalts aus einem Vergleich der beiden Restgehalte an Sauerstoff (ROC(M), ROC(R)).

2. Verfahren nach Anspruch 1, **dadurch gekennzeichnet, dass** ein erster Teilstrom (3, 3a, 3c) über eine Abreicherungseinrichtung (9, 9a, 9c) für Teer und ein zweiter Teilstrom (4, 4a, 4c) unter Umgehung der Abreicherungseinrichtung (9, 9a, 9c) geführt werden und die Differenz zwischen den Restgehalten an Sauerstoff (ROC(M), ROC(R)) der beiden Teilströme (3, 3a, 3c, 4, 4a, 4c) ermittelt wird.

3. Verfahren nach Anspruch 2, **dadurch gekennzeichnet, dass** die Restgehalte an Sauerstsoff (ROC(M), ROC(R)) in zwei identischen Messaufbauten (5, 6) mit Ausnahme einer wirksamen Abreicherungseinrichtung (9) in einem der Messaufbauten (5, 6) ermittelt werden.

4. Verfahren nach Anspruch 2, **dadurch gekennzeichnet, dass** die Restgehalte an Sauerstoff (ROC(M), ROC(R)) in einem einzigen Messaufbau (5a, 5c) zeitlich alternierend in ersten Zeittakten mittels eines über die Abreicherungseinrichtung (9a, 9c) geführten Teilstroms (3a, 3c) und in zweiten mit den ersten Zeittakten abwechselnden Zeittakten mittels eines an der Abreicherungseinrichtung (9a, 9c) vorbeigeführten Teilstroms (4a, 4c) ermittelt werden.

5. Verfahren nach einem der Ansprüche 2 bis 4, **dadurch gekennzeichnet, dass** dem Sauerstoffsensor (18, 18a, 19) ein Katalysator (15, 15a, 16) zur Umsetzung aller in dem zumindest einen Teilstrom (3, 4) vorhandenen oxidierbaren Komponenten vorgeschaltet wird.

6. Verfahren nach einem der Ansprüche 2 bis 4, **dadurch gekennzeichnet, dass** eine Umsetzung der in einem von dem zumindest einen Teilstrom (23a, 3b, 3c, 4c) abgetrennten Arbeitsbereich eines als Lambdasonde ausgebildeten Sauerstoffsensors (18a, 18b, 18c) vorhandenen oxidierbaren Komponenten mittels der Lambdasonde durchgeführt wird.

7. Verfahren nach Anspruch 1, **dadurch gekennzeichnet, dass** in einem einzigen Teilstrom (3b) mittels einer von einem Sauerstoffsensor (24b), insbesondere einer Lambdasonde gesteuerten Dosiereinrichtung (10b) ein vorgegebener Sauerstoffgehalt in dem Teilstrom (3b) eingestellt wird, wobei der Teilstrom (3b) durch die Abreicherungseinrichtung (9b) geführt wird und anschließend an einem als Lambdasonde ausgebildeten Sauerstoffsensor (18b) unter Umsetzung der in einen mit dem Teilstrom (3b) verbundenen Arbeitsbereich der Lambdasonde überführten oxidierbaren Komponenten der Restgehalt an Sauerstoff (ROC(R)) ermittelt und der Teergehalt mittels eines Vergleichs einer zuvor ermittelten Kalibrierkurve des Teergehalts über den Restgehalt an Sauerstoff (ROC(R)) bestimmt wird.

8. Verfahren nach Anspruch 1, **dadurch gekennzeichnet, dass** zwei Teilströme (3c, 4c) mittels wechselweiser Taktung des Messgasgemischs (2c) erzeugt werden und ein erster Teilstrom (4c) an der Abreicherungseinrichtung (9c) unter Beibehaltung des Teergehalts vorbeigeführt und ein zweiter Teilstrom (3c) über die Abreicherungseinrichtung (9c) unter Abreicherung des Teergehalts geführt werden, wobei den teerhaltigen Teilströmen (3c, 4c) mittels eines ersten Sauerstoffsensors (18c) geregelt ein vorgegebener Sauerstoffgehalt zugeführt wird und die Restsauerstoffgehalte (ROC(M), ROC(R)) mit nicht abgereichertem und abgereichertem Teergehalt mittels eines zweiten, als Lambdasonde mit einem von den Teilströmen (3c, 4c) abgetrennten Arbeitsbereich ausgeführten Sauerstoffsensors (18c) unter vollständiger Umsetzung der im Arbeitsbereich enthaltenen oxidierbaren Komponenten ermittelt werden und anhand eines Vergleichs der Restgehalte (ROC(R), ROC(M)) der Teergehalt ermittelt wird.

9. Verfahren nach Anspruch 8, **dadurch gekennzeichnet, dass** als erster und als zweiter Sauerstoffsensor (18c) derselbe, als Lambdasonde ausgebildete Sauerstoffsensor (18c) verwendet wird.

10. Verfahren nach einem der Ansprüche 7 bis 9, **dadurch gekennzeichnet, dass** mittels des den Sauerstoffgehalt regelnden Sauerstoffsensors (24b, 18c) in dem Teilstrom (3b, 3c, 4c) eine Luftzahl gleich eins eingestellt wird.

11. Messsystem (1, 1a, 1b, 1c) zur Durchführung des Verfahrens gemäß den Ansprüchen 1 bis 10 mit einem Messaufbau (5, 5a, 5b, 5c) mit einer zumindest einen Teilstrom (3, 3a, 3b, 3c, 4, 4c, 23a) eines Messgasgemisches (2, 2a, 2b, 2c) fördernden Pumpe (7, 7b, 7c), mit einer Dosiereinrichtung (10, 10a, 10b, 10c) für ein Reaktionsgas mit vorgegebenem Sauerstoffgehalt, einer Abreicherungseinrichtung (9, 9a, 9b, 9c) für in dem Messgasgemisch (2, 2a, 2b, 2c) enthaltenem Teergehalt, einer Umsetzungseinrichtung zur Umsetzung eines vorgegebenen Anteils in dem zumindest einen Teilstrom (3, 3a, 3b, 4, 4c, 23a) vorhandenen oxidierbaren Komponenten, einem Sauerstoffsensor (18, 18a, 18b, 18c) zur Ermittlung des von der Abreicherung des Teergehalts abhängigen ersten Restgehalts an Sauerstoff (ROC(R)) und des zweiten Restgehalts an Sauerstoff (ROC(M)) und einer Auswerteeinrichtung eingerichtet zur Ermittlung eines
Summenparameters des Teergehalts aus einem Vergleich der beiden Restgehalte an Sauerstoff (ROC(M), ROC(R)).

12. Messsystem (1) nach Anspruch 11, **dadurch gekennzeichnet, dass** dem Messaufbau (5) ein zweiter, bis auf eine fehlende Abreicherungseinrichtung identischer Messaufbau (6) parallelgeschaltet ist.

13. Messsystem (1b, 1c) nach Anspruch 11, **dadurch gekennzeichnet, dass** zur Einstellung des vorgegebenen Sauerstoffgehalts hinter der Abreicherungseinrichtung (9b, 9c) in dem zumindest einen Teilstrom (3b, 3c, 4c) ein die Dosiereinrichtung (10b, 10c) steuernder Sauerstoffsensor (24b, 18c), insbesondere eine Lambdasonde vorgesehen ist.

14. Messsystem (1c) nach Anspruch 13, **dadurch gekennzeichnet, dass** der Sauerstoffsensor (18c) zur Erfassung der Restgehalte an Sauerstoff (ROC(R), ROC(M)) zusätzlich zur Einstellung des Sauerstoffgehalts in den Teilströmen (3c, 4c) mit und ohne Abreicherung des Teergehalts mittels der Dosiereinrichtung (10c) vorgesehen ist.

15. Messsystem (1b, 1c) nach einem der Ansprüche 11 bis 14, **dadurch gekennzeichnet, dass** zur Erfassung der Restgehalte an Sauerstoff (ROC(R), ROC(M)) und gegebenenfalls zur Dosierung des Sauerstoffgehalts in den zumindest einen Teilstrom (3, 3a, 3b, 3c, 4, 4c, 23a) eine Lambdasonde mit Schmalbandcharakteristik zur Erfassung eines engen Konzentrationsbereichs des Sauerstoffgehalts um den Lambdawert gleich eins und/oder eine Lambdasonde mit Breitbandcharakteristik zur Erfassung eines um mindestens eine Größenordnung gegenüber dem Lambdawert gleich eins erweiterten Konzentrationsbereichs des Sauerstoffgehalts vorgesehen ist.

## Claims

1. Method for detecting a tar content in a measuring gas mixture (2, 2a, 2b, 2c) containing oxidisable components, including at least the following method steps:

   - removing at least one partial stream (3, 3a, 3b, 3c, 4, 4c, 23a) of the measuring gas mixture (2, 2a, 2b, 2c),
   - adding a predetermined content of oxygen to the at least one partial stream (3, 3c, 4, 4c, 23a),
   - changing the oxygen content by converting a predetermined amount of oxidisable components present in the at least one partial stream (3, 3a, 3b, 3c, 4, 4c, 23a) to residual contents of oxygen ROC(R), ROC(M)),
- determining a first residual oxygen content (ROC(R)), which is dependent on a depletion of the tar content performed by means of a depletion device (9, 9a, 9b), by means of at least one oxygen sensor (18, 18a, 18b, 18c, 19),
- determining a second residual oxygen content (ROC(M)) by means of at least one oxygen sensor (18, 18a, 18b, 18c, 19) without depleting the tar content,
- determining a sum parameter of the tar content from a comparison of the two residual oxygen contents (ROC(M), ROC(R)).

2.  Method according to claim 1, **characterised in that** a first partial stream (3, 3a, 3c) is passed via a depletion device (9, 9a, 9c) for tar and a second partial stream (4, 4a, 4c) is passed bypassing the depletion device (9, 9a, 9c) and the difference between the residual oxygen contents (ROC(M), ROC(R)) of the two partial streams (3, 3a, 3c, 4, 4a, 4c) is determined.

3.  Method according to claim 2, **characterised in that** the residual oxygen contents (ROC(M), ROC(R)) are determined in two identical measuring arrangements (5, 6) with the exception of an effective depletion device (9) in one of the measuring arrangements (5,6).

4.  Method according to claim 2, **characterised in that** the residual oxygen contents (ROC(M), ROC(R)) are determined in a single measurement arrangement (5a, 5c) alternating in time in first time cycles by means of a partial stream (3a, 3c) passed via the depletion device (9a, 9c) and in second time cycles alternating with the first time cycles by means of a partial stream (4a, 4c) passed via the depletion device (9a, 9c).

5.  Method according to any of claims 2 to 4, **characterised in that** a catalyst (15, 15a, 16) for converting all oxidisable components present in the at least one partial stream (3, 4) is connected upstream of the oxygen sensor (18, 18a, 19).

6.  Method according to any of claims 2 to 4, **characterised in that** a conversion of the oxidisable components present in a working area, separate from the at least one partial stream (23a, 3b, 3c, 4c), of an oxygen sensor (18a, 18b, 18c) designed as a lambda probe, is carried out by means of the lambda probe.

7.  Method according to claim 1, **characterised in that** in a single partial stream (3b) by means of a metering device (10b) controlled by an oxygen sensor (24b), in particular a lambda probe, a predetermined oxygen content is set in the partial stream (3b), wherein the partial stream (3b) is passed through the depletion device (9b) and then the residual oxygen content (ROC(R)) is determined at an oxygen sensor (18b) designed as a lambda probe, with conversion of the oxidisable components transferred into a working area of the lambda probe connected to the partial stream (3b), and the tar content is determined by means of a comparison of a previously determined calibration curve of the tar content with respect to the residual oxygen content (ROC(R)).

8.  Method according to claim 1, **characterised in that** two partial streams (3c, 4c) are generated by means of alternating clocking of the measuring gas mixture (2c) and a first partial stream (4c) is led past the depletion device (9c) while retaining the tar content and a second partial stream (3c) is passed via the depletion device (9c) with depletion of the tar content, wherein a predetermined oxygen content is supplied to the tar-containing partial streams (3c, 4c) in a controlled manner by means of a first oxygen sensor (18c) and the residual oxygen contents (ROC(M), ROC(R)) with non-depleted and depleted tar content are determined by means of a second oxygen sensor (18c) designed as a lambda probe with a working area separate from the partial streams (3c, 4c) with complete conversion of the oxidisable components contained in the working area, and the tar content is determined on the basis of a comparison of the residual contents (ROC(R), ROC(M)).

9.  Method according to claim 8, **characterised in that** the same oxygen sensor (18c), designed as a lambda probe, is used as the first and second oxygen sensor (18c).

10. Method according to any of claims 7 to 9, **characterised in that** a lambda value equal to one is set in the partial stream (3b, 3c, 4c) by means of the oxygen sensor (24b, 18c) controlling the oxygen content.

11. Measuring system (1, 1a, 1b, 1c) for performing the method according to claims 1 to 10 having a measuring arrangement (5, 5a, 5b, 5c) with a pump (7, 7b, 7c) conveying at least one partial stream (3, 3a, 3b, 3c, 4, 4c, 23a) of a measuring gas mixture (2, 2a, 2b, 2c), having a metering device (10, 10a, 10b, 10c) for a reaction gas with a predetermined oxygen content, a depletion device (9, 9a, 9b, 9c) for tar content contained in the measuring gas mixture (2, 2a, 2b, 2c), a conversion device for converting a predetermined proportion of oxidisable components present in the at least one partial stream (3, 3a, 3b, 4, 4c, 23a), an oxygen sensor (18, 18a, 18b, 18c) for determining the first residual oxygen content (ROC(R)) dependent on the depletion of the tar content and the second residual oxygen content (ROC(M)) and an evaluation device set up for determining a sum parameter of the tar content from a comparison of the two residual oxygen contents (ROC(M)), ROC(R)).

12. Measuring system (1) according to claim 11, **characterised in that** a second measuring arrangement (6) is connected in parallel with the measuring arrangement (5) which is identical except for a missing depletion device.

13. Measuring system (1b, 1c) according to claim 11, **characterised in that** an oxygen sensor (24b, 18c), in particular a lambda probe, controlling the metering device (10b, 10c) is provided for setting the predetermined oxygen content downstream of the depletion device (9b, 9c) in the at least one partial stream (3b, 3c, 4c).

14. Measuring system (1c) according to claim 13, **characterised in that** the oxygen sensor (18c) for detecting the residual oxygen contents (ROC(R), ROC(M)) is provided in addition to setting the oxygen content in the partial streams (3c, 4c) with and without depletion of the tar content by means of the metering device (10c).

15. Measuring system (1b, 1c) according to any of claims 11 to 14, **characterised in that** for detecting the residual oxygen contents (ROC(R), ROC(M)) and if necessary for metering the oxygen content into the at least one partial stream (3, 3a, 3b, 3c, 4, 4c, 23a) a lambda probe with a narrow-band characteristic is provided for detecting a narrow concentration range of the oxygen content around the lambda value equal to one and/or a lambda probe with a broadband characteristic is provided for detecting a concentration range of the oxygen content which is broader by at least one order of magnitude compared to the lambda value equal to one.

**Revendications**

1. Procédé permettant de détecter une teneur en goudron dans un mélange gazeux à mesurer (2, 2a, 2b, 2c) contenant des composants oxydables, comprenant au moins les étapes de procédé suivantes :

   - extraire au moins un flux partiel (3, 3a 3b, 3c, 4, 4c, 23a) du mélange gazeux à mesurer (2, 2a, 2b, 2c),
   - ajouter une dose d'une teneur prédéterminée en oxygène audit au moins un flux partiel (3, 3c, 4, 4c, 23a),
   - modifier la teneur en oxygène par la conversion d'une quantité prédéterminée de composants oxydables présents dans ledit au moins un flux partiel (3, 3a, 3b, 3c, 4, 4c, 23a) en teneurs résiduelles en oxygène ROC(R), ROC(M)),
   - déterminer une première teneur résiduelle en oxygène (ROC(R)), qui dépend d'un appauvrissement de la teneur en goudron effectué au moyen d'un dispositif d'appauvrissement (9, 9a, 9b), au moyen d'au moins un capteur d'oxygène (18, 18a, 18b, 18c, 19),
   - déterminer une deuxième teneur résiduelle en oxygène (ROC(M)) au moyen d'au moins un capteur d'oxygène (18, 18a, 18b, 18c, 19) sans appauvrissement de la teneur en goudron
   - déterminer un paramètre de somme de la teneur en goudron à partir d'une comparaison des deux teneurs en oxygène résiduel (ROC(M), ROC(R)).

2. Procédé selon la revendication 1, **caractérisé en ce qu'**un premier flux partiel (3, 3a, 3c) est guidé via un dispositif d'appauvrissement (9, 9a, 9c) pour le goudron et un deuxième flux partiel (4, 4a, 4c) est guidé en contournant le dispositif d'appauvrissement (9, 9a, 9c), et la différence entre les teneurs résiduelles en oxygène (ROC(M), ROC(R)) des deux flux partiels (3, 3a, 3c, 4, 4a, 4c) est déterminée.

3. Procédé selon la revendication 2, **caractérisé en ce que** les teneurs en oxygène résiduel (ROC(M), ROC(R)) sont déterminées dans deux installations de mesure identiques (5, 6) à l'exception d'un dispositif d'appauvrissement effectif (9) dans l'une des installations de mesure (5, 6).

4. Procédé selon la revendication 2, **caractérisé en ce que** les teneurs résiduelles en oxygène (ROC(M), ROC(R)) sont déterminées dans une seule installation de mesure (5a, 5c) en alternant dans le temps dans des premiers cycles temporels au moyen d'un flux partiel (3a, 3c) guidé via le dispositif d'appauvrissement (9a, 9c) et dans des deuxièmes cycles temporels alternant avec les premiers cycles temporels au moyen d'un flux partiel (4a, 4c) guidé devant le dispositif d'appauvrissement (9a, 9c).

**5.** Procédé selon l'une quelconque des revendications 2 à 4, **caractérisé en ce qu'**un catalyseur (15, 15a, 16) est monté en amont du capteur d'oxygène (18, 18a, 19) pour convertir tous les composants oxydables présents dans ledit au moins un flux partiel (3, 4).

**6.** Procédé selon l'une des revendications 2 à 4, **caractérisé en ce qu'**une conversion des composants oxydables présents dans une plage de travail d'une sonde à oxygène (18a, 18b, 18c) réalisée sous la forme de sonde lambda, qui est séparée dudit au moins un flux partiel (23a, 3b, 3c, 4c), est effectuée au moyen de la sonde lambda.

**7.** Procédé selon la revendication 1, **caractérisé en ce qu'**une teneur en oxygène prédéterminée est réglée dans un seul flux partiel (3b) au moyen d'un dispositif de dosage (10b) commandé par une sonde à oxygène (24b), en particulier une sonde lambda, le flux partiel (3b) étant guidé à travers le dispositif d'appauvrissement (9b) et la teneur résiduelle en oxygène (ROC(R)) étant ensuite déterminée au niveau d'un capteur d'oxygène (18b) réalisé sous la forme de sonde lambda, en convertissant les composants oxydables transférés dans une plage de travail de la sonde lambda raccordée au flux partiel (3b), et la teneur en goudron étant déterminée au moyen d'une comparaison d'une courbe d'étalonnage, déterminée au préalable, de la teneur en goudron sur la teneur résiduelle en oxygène (ROC(R)).

**8.** Procédé selon la revendication 1, **caractérisé en ce que** deux flux partiels (3c, 4c) sont générés au moyen d'un cadencement alterné du mélange gazeux à mesurer (2c), et un premier flux partiel (4c) est guidé devant le dispositif d'appauvrissement (9c) tout en maintenant la teneur en goudron et un deuxième flux partiel (3c) est guidé via le dispositif d'appauvrissement (9c) tout en appauvrissant la teneur en goudron, une teneur en oxygène prédéterminée étant apportée de manière contrôlée aux flux partiels (3c, 4c) contenant du goudron au moyen d'un premier capteur d'oxygène (18c), et les teneurs résiduelles en oxygène (ROC(M), ROC(R)) avec une teneur en goudron non appauvrie et appauvrie sont mesurées au moyen d'un deuxième capteur d'oxygène (18c), qui est réalisé sous la forme d'une sonde lambda avec une plage de travail séparée des flux partiels (3c, 4c), 4c), et la teneur en goudron est déterminée sur la base d'une comparaison des teneurs résiduelles (ROC(R), ROC(M)).

**9.** Procédé selon la revendication 8, **caractérisé en ce que** la même sonde à oxygène (18c), qui est réalisée sous la forme de sonde lambda, est utilisée comme première et comme deuxième sonde à oxygène (18c).

**10.** Procédé selon l'une quelconque des revendications 7 à 9, **caractérisé en ce qu'**un coefficient d'air égal à un est réglé dans le flux partiel (3b, 3c, 4c) au moyen du capteur d'oxygène (24b, 18c) contrôlant la teneur en oxygène.

**11.** Système de mesure (1, 1a, 1b, 1c) pour la mise en œuvre du procédé selon les revendications 1 à 10, comportant une installation de mesure (5, 5a 5b, 5c) avec une pompe (7, 7b, 7c) transportant au moins un flux partiel (3, 3a, 3b, 3c, 4, 4c, 23a) d'un mélange gazeux à mesurer (2, 2a, 2b, 2c), avec un dispositif de dosage (10, 10a, 10b, 10c) pour un gaz de réaction avec une teneur en oxygène prédéterminée, un dispositif d'appauvrissement (9, 9a, 9b, 9c) pour la teneur en goudron contenue dans le mélange gazeux à mesurer (2, 2a, 2b, 2c), un dispositif de conversion pour convertir une part prédéterminée de composants oxydables présents dans ledit au moins un flux partiel (3, 3a, 3b, 4, 4c, 23a), un capteur d'oxygène (18, 18a, 18b, 18c) pour déterminer la première teneur résiduelle en oxygène (ROC(R)), qui dépend de l'appauvrissement de la teneur en goudron, et la deuxième teneur résiduelle en oxygène (ROC(M)), et un dispositif d'évaluation conçu pour déterminer un paramètre de somme de la teneur en goudron à partir d'une comparaison des deux teneurs en oxygène résiduel (ROC(M), ROC(R)).

**12.** Système de mesure (1) selon la revendication 11, **caractérisé en ce qu'**une deuxième installation de mesure (6), qui est identique à l'exception d'un dispositif d'appauvrissement manquant, est montée en parallèle avec l'installation de mesure (5).

**13.** Système de mesure (1b, 1c) selon la revendication 11, **caractérisé en ce qu'**un capteur d'oxygène (24b, 18c) commandant le dispositif de dosage (10b, 10C), en particulier une sonde lambda, est prévu pour régler la teneur en oxygène prédéterminée en aval du dispositif d'appauvrissement (9b, 9c) dans ledit au moins un flux partiel (3b, 3c, 4c).

**14.** Système de mesure (1c) selon la revendication 13, **caractérisé en ce que** le capteur d'oxygène (18c) pour détecter les teneurs résiduelles en oxygène (ROC(R), ROC(M)) est prévu en plus du réglage de la teneur en oxygène dans les flux partiels (3c, 4c) avec et sans appauvrissement de la teneur en goudron au moyen du dispositif de dosage (10c).

**15.** Système de mesure (1b, 1c) selon l'une des revendications 11 à 14, **caractérisé en ce que** pour détecter la teneur résiduelle en oxygène (ROC(R), ROC(M)) et, le cas échéant, pour doser la teneur en oxygène dans ledit au moins un flux partiel (3, 3a, 3b, 3c, 4, 4c, 23a), une sonde lambda à caractéristique à bande étroite est prévue pour détecter une plage de concentration étroite de la teneur en oxygène autour de la valeur lambda égale à un et/ou une sonde lambda à caractéristique à large bande est prévue pour détecter une plage de concentration de la teneur en oxygène qui est étendue d'au moins un ordre de grandeur par rapport à la valeur lambda égale à un.

Fig. 1

Fig. 2

Fig. 3

1c

3c

2c

9c

29c

28c

18c

25c  4c  26c

7c

10c

27c

12c  5c

Fig. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102013204463 A1

**In der Beschreibung aufgeführte Nicht-Patentliteratur**

- **DRUCKSCHRIFT MOERSCH, 0. ; SPLIETHOFF, H. ; HEIN, K. R. G.** Tar quantification with a new online analyzing method. *Biomass and Bioenergy,* 2000, 79-86